# EUROPEAN PATENT SPECIFICATION

(11) **EP 0 471 334 B1**
(45) Date of publication and mention of the grant of the patent: **18.06.1997**
(21) Application number: 91113567.1
(22) Date of filing: 13.08.1991
(51) Int. Cl.: A61B 17/064

(54) **Absorbable surgical fastener with bone penetrating elements**
Absorbierbare, chirurgische Befestigungsvorrichtung mit knochendurchdringenden Elementen
Dispositif résorbable de fixation, avec éléments pénétrant dans un os

(30) Priority: 13.08.1990 US 566242
(43) Date of publication of application: 19.02.1992
(73) Proprietor: United States Surgical Corporation, Norwalk, Connecticut 06856 (US)
(72) Inventor: Sander, Thomas W., Winona Lake, Indiana 46590 (US); White, Jeffrey S., Ridgefield, Connecticut 06877 (US)
(74) Representative: Marsh, Roy David

(56) References cited:
- EP-A- 0 346 033
- EP-A- 0 390 613
- WO-A-87/01595
- DE-A- 2 721 075
- US-A- 4 838 264

## Description

### BACKGROUND OF THE INVENTION

### 1. Field of the Invention

This invention relates to surgical fasteners for fastening body tissue and more particularly relates to an at least partially absorbable fastener for fastening bone or hard tissue. Such fasteners are disclosed in EP-A-0 346 033.

### 2. Background of the Related Art

Bone fastening or fixation devices are well known in the art. Typically, such fasteners are in the form of staples, pins, screws, and wires. For example, both Pratt et al., U.S. Patent No. 4,454,875 and Ellison et al., U.S. Patent No. 4,570,623 disclose staples for being driven into bones. Staples of this type are generally fabricated of biologically inert metal, such as stainless steel, titanium, cobalt-chromium-molybdenum alloys and the like. The staple must be relatively strong and hard so that it can be easily driven directly into bone or hard tissue.

Other metal fasteners are well known commercial products used for a wide variety of bone fixation procedures. Pins and wires are generally constructed from stainless steel and are grasped in a drill chuck and self-drilled directly into bone to treat a given traumatic or pathological condition.

The disadvantage of metal fasteners is that after they have completed their function of supporting the bone while the bone heals, they remain permanently in the body. Problems can arise after healing, for example, by corrosion of the metal, or when the pins or staples work loose from their moorings and migrate through body tissue.

Furthermore, permanent metal fixation devices shield the bone from beneficial stresses after healing. It has been shown that moderate periodic stress on bone tissue, such as the stress produced by exercise, helps to prevent decalcification of the bone. Under some conditions, the stress shielding which results from the long term use of metal bone fixation devices can lead to osteoporosis.

These disadvantages can be mitigated by the use of bioabsorbable surgical fasteners, which degrade over a period of time thereby gradually transferring more support load to the bone as it heals. Such fasteners for bone are also known in the art. For instance, Tunc, U.S. Patent No. 4,539,981 teaches the use of polymers of L(-)lactide for fabricating bone fixation devices. Moreover, various types of bioabsorbable pin fasteners have been commercialized. For example, some types of pins are fabricated from poly (p-dioxanone) and are indicated for use to fix in place small bony fragments in the knee and hand, where such fragments are not in tension. As is characteristic for all such absorbable pins, holes must be previously drilled into the bone in order for the pins to be inserted. Bioabsorbable fasteners are not self-inserting, i.e. they are not capable of being driven or screwed directly into bone because the polymeric material they are made of is relatively soft. The necessity to predrill holes in the injured bone adds to the surgical procedures and lengthens the time required to complete the operation.

EP-A-0 346 033 discloses a bone pin made with a tapered polymeric portion and a cutting device secured to the smaller end of the taper.

DE-A-2 721 075 discloses a bone nail of special alloy with a hard steel drilling tip.

US-A-4 838 264 discloses a pin with a bone-penetrating tip of titanium or alumina ceramic mounted to the distal end of a screw-threaded shank. To the other end of the shank is mounted a driving head with a slot for a screw driving blade. The pin is used within a device for holding the skull of a patient in a fixed position during surgery or healing.

### SUMMARY OF THE INVENTION

The present invention, defined in claim 1 below, is a step forward from the fastener described in EP-A-0 390 613, which falls under Article 54(3) EPC, which was not published until 03 October 1990, later than the US priority date of 13 August 1990 declared in the present application.

The bone-penetrating element of the surgical fastener is of sufficient length to be gripped by a drilling means and inserted into bone or hard tissue. The bioabsorbable fastening body portion is uniform in cross-section, i.e., it is not tapered.

### BRIEF DESCRIPTION OF THE DRAWINGS

Fig. 1 illustrates an embodiment of the pin fastener of the present invention.

Fig. 2 illustrates a double tipped embodiment of the pin fastener of the present invention.

### DETAILED DESCRIPTION OF THE INVENTION

The basis of the present invention is the attachment of bone-penetrating elements to bioabsorbable polymer implant devices, such as fasteners, thereby enabling the implant devices to be drilled or driven directly into bone or other hard tissue.

The fastening body portion of the bone fixation device of the present invention is fabricated from a biodegradable material such as one or more of the several types of bioabsorbable polymers commonly used in such applications. Examples include poly (p-dioxanone), polylactide, polyglycolides, polycaprolactone, poly (orthoesters) and trimethylene carbonate polymer and the like, as well as copolymers and/or mixtures and blends of the same. Optionally, the biodegradable material used in the various embodiments of this invention, may contain reinforcing fibers, so as to produce a high strength composite. The reinforcing fibers can be, for example, polymeric, or ceramic materials, and either bioabsorbable or permanent.

The terms "biodegradable" and "bioabsorbable" are used interchangeably herein, and refer to materials which are chemically broken down and/or assimilated by human (or animal) body tissue.

The bone penetrating elements of the fastener of the present invention are preferably in the form of relatively hard tips for initially contacting the bone and enabling the fastener to penetrate the bone when a suitable driving force is applied. The tips preferably have means for cutting bone or hard tissue such as a relatively sharp point or one or more sharp edges.

The tips should be of sufficient size and mass relative to the fastening body portion of the surgical fastener, to have the mechanical strength necessary to penetrate bone or hard tissue. However, because the material best suited for fabricating the tips is non-biodegradable, the optimum size of the tips is the minimum size necessary to perform its function of penetrating hard tissue and bone for those surgical applications in which the tips will remain embedded in the bone. As discussed below, not all surgical applications require the bone penetrating tips to remain embedded in bone.

The bone-penetrating element may be fabricated from a ceramic material which can be selected from zirconia alumina and carbon/carbon composites. Alternatively, the bone-generating element may be fabricated from a metal, which may be stainless steel, titanium alloy or cobalt-chromium molybdenum alloy.

As can readily be seen, the method for using the present invention to fasten segments of bone or hard tissue is relatively simple. Being provided with the surgical fastener of the present invention, the surgeon implants the device into bone or hard tissue so that the bioabsorbable fastening body portion holds and maintains the segments of bone or hard tissue in close adjacency for sufficient period of time to promote healing. The fasteners will degrade over a period of time leaving only the metal tips which, being small, are far less intrusive, and have little tendency to work loose from the bone and migrate.

In some surgical methods employing the fasteners of the present invention no hard nonabsorbable piece at all remains. For example, the pin can be driven completely across a fracture site so that the bone penetrating element emerges from the far side of the bone. In such a case, the bone penetrating tip will be cut off and removed, leaving no portion of the fastener which cannot be absorbed.

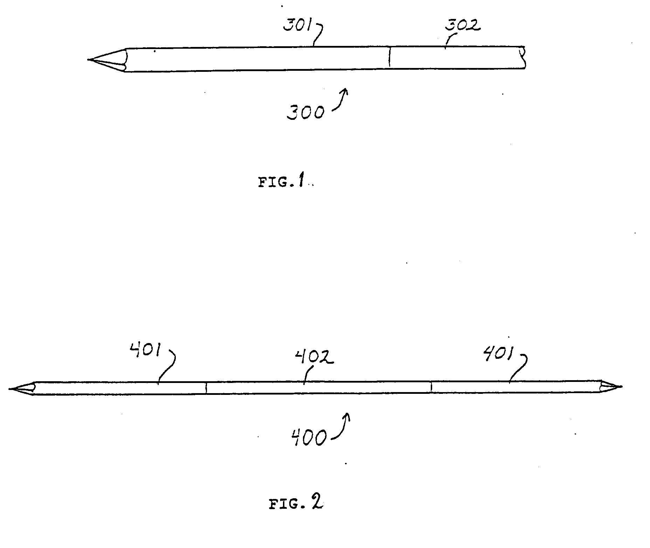

In a specific embodiment 300 of the present invention illustrated in Fig. 1 the bone piercing element 301 is of sufficient length such that it can be gripped by a drilling device and inserted into bone or hard tissue. The application of a drilling force to the bone penetrating element 301 rather than the absorbable fastener portion or shaft 302 prevents deformation of the relatively softer absorbable fastener portion 302 because it is not subjected to the torsional stress of twisting. The bone penetrating tip 301 is from 7.5 to 20 cm (3 inches to 8 inches) in length. The bioabsorbable shaft portion 302 is from 5 to 10 cm (2 to 4 inches) in length. Fastener 300 is substantially uniform in cross-section, i.e. it is not tapered. Uniformity of cross section provides better fitting of the fastener in the bone.

For some surgical applications gripping or drilling may need to be done from both ends of a pin. For example, in operations where a surgeon is required to insert a pin through a first bone fragment and into a second bone fragment across a fracture site, it may happen that the surgeon is satisfied with the positioning of the pin in the second bone fragment but not the first. With a single tipped pin, the surgeon would have to remove the pin altogether, and select a preferred location through the first bone fragment. However, it would be very difficult for the surgeon then to line up the new placement of the pin in the first fragment with the previously made hole in the second fragment.

Fig. 2 illustrates a pin 400 having a bone penetrating element 401 at both ends of a bioabsorbable fastener portion or shaft 402. Pin fastener 400 is preferably substantially uniform in cross section. The bone penetrating element 401 of this embodiment is preferably from 7.5 to 12.5 cms (3 to 5 inches) in length, and the bioabsorbable center portion 402 is from 5 to 10 cm (2 to 4 inches) in length. The tips and shafts can be attached to each other by swaging, crimping, screw fitting, or other methods which one skilled in the art can envision.

With this double tipped pin, the surgeon would pull the pin through the second fragment until the second penetrating element clears the first bone fragment. The surgeon would reapply the pin through the first fragment by means of the second bone penetrating tip.

The tips 301 and 401 are fabricated from material which has hardness and strength suitable for its purpose of drilling bone. Such materials have been mentioned above and include ceramics and implant grade metal elements and alloys. The bioabsorbable shaft portions are fabricated from any suitable bioabsorbable compound such as those mentioned above.

## Claims

1. A surgical fastener (200) comprising a bioabsorbable shaft (203) of uniform cross-section and at least one elongate bone-penetrating element (201) adapted to be gripped by a drilling means and inserted into bone, the bone-penetrating element being attached to one end of the shaft and aligned with the shaft, the length of the bone-penetrating element being in a range of from 7.5 to 20 cms and the length of the shaft being from 5 to 10 cms.

2. A fastener as claimed in claim 1 wherein said bioabsorbable shaft is fabricated from a polymeric material.

3. A fastener as claimed in claim 1 or 2 wherein said bone-penetrating element is fixed to the shaft by swaging or crimping.

4. A fastener as claimed in claim 1, 2 or 3 wherein said bone-penetrating element is fabricated from a ceramic material.

5. A fastener as claimed in claim 4 wherein the ceramic material comprises a material selected from zirconia, alumina, and carbon/carbon composites.

6. A fastener as claimed in claim 1, 2 or 3 wherein said bone-penetrating element is fabricated from a metal.

7. A fastener as claimed in claim 6 wherein said metal is selected from stainless steel, titanium, titanium alloy, and cobalt-chromium-molybdenum alloy.

8. A fastener as claimed in any one of the preceding claims wherein said bioabsorbable shaft is fabricated from a bioabsorbable polymer selected from poly(p-dioxanone), polylactide, polyglycolide, polycaprolactone, poly(orthoesters), and trimethylene carbonate polymer, and copolymers and/or mixtures thereof.

9. A fastener as claimed in any one of the preceding claims wherein said bioabsorbable shaft and the bone-penetrating element are screw-fitted together.

10. A fastener as claimed in any one of the preceding claims wherein the bioabsorbable shaft has a said bone-penetrating element attached to each of its ends.

11. A fastener as claimed in claim 10 wherein said bone-penetrating elements are each from about 7.5 to 12.5cms (3 to 5 inches) in length.

## Patentansprüche

1. Chirurgischer Befestiger (200) umfassend einen bioabsorbierbaren Schaft (203) von gleichmäßigem Querschnitt und zumindest ein langgestrecktes knochendurchdringendes Element (201), das dazu geeignet ist, von einem Bohrmittel gegriffen und in Knochen eingeführt zu werden, wobei das knochendurchdringende Element an einem Ende des Schaftes angebracht ist und mit dem Schaft ausgerichtet ist und die Länge des knochendurchdringenden Elements im Bereich von 7,5 bis 20 cm ist und die Länge des Schaftes von 5 bis 10 cm ist.

2. Befestiger gemäß Anspruch 1, wobei der bioabsorbierbare Schaft aus einem polymerischen Material hergestellt ist.

3. Befestiger gemäß Anspruch 1 oder 2, wobei das knochendurchdringende Element an dem Schaft durch Gesenkschmieden oder Umbiegen befestigt ist.

4. Befestiger gemäß Anspruch 1, 2 oder 3, wobei das knochendurchdringende Element aus einem keramischen Material hergestellt ist.

5. Befestiger gemäß Anspruch 4, wobei das keramische Material umfaßt ein Material, das aus Zirkonium, Aluminiumoxyd und Kohlenstoff/Kohlenstoffverbundstoffen ausgewählt ist.

6. Befestiger gemäß Anspruch 1, 2 oder 3, wobei das knochendurchdringende Element aus einem Metall hergestellt ist.

7. Befestiger gemäß Anspruch 6, wobei das Metall von Edelstahl, Titan, Titanlegierung, und Kobalt-Chrom-Molybdenlegierung ausgewählt wird.

8. Befestiger gemäß einem der vorhergehenden Ansprüche, wobei der bioabsorbierbare Schaft aus einem bioabsorbierbaren Polymer hergestellt ist, das ausgewählt ist aus Poly(p-Dioxanon), Polylactid, Polyglycolid, Polycaprolacton, Poly(orthoestern) und Trimethylencarbonatpolymer und Copolymeren und/oder Mischungen derselben.

9. Befestiger gemäß einem der vorhergehenden Ansprüche, wobei der bioabsorbierbare Schaft und das knochendurchdringende Element miteinander verschraubt sind.

10. Befestiger gemäß einem der vorhergehenden Ansprüche, wobei der bioabsorbierbare Schaft ein knochendurchdringendes Element an jedem seiner Enden angebracht hat.

11. Befestiger gemäß Anspruch 10, wobei die knochendurchdringenden Elemente beide von etwa 7,5 bis 12,5 cm (3 bis 5 inches) lang sind.

## Revendications

1. Attache chirurgicale (200) comportant une tige biorésorbable (203) de section transversale uniforme et au moins un élément oblong (201) pénétrant dans l'os apte à être saisi par un moyen de forage et inséré dans l'os, l'élément pénétrant dans l'os étant fixé à une extrémité de la tige et aligné avec la tige, la longueur de l'élément pénétrant dans l'os se situant dans une plage de 7,5 à 20 cm, et la longueur de la tige allant de 5 à 10 cm.

2. Attache selon la revendication 1, où ladite tige biorésorbable est fabriquée en polymère.

3. Attache selon la revendication 1 ou 2, où ledit élément pénétrant dans l'os est fixé à la tige par estampage ou sertissage.

4. Attache selon la revendication 1, 2 ou 3, où ledit élément pénétrant dans l'os est fabriqué en une céramique.

5. Attache selon la revendication 4, où la céramique comprend un matériau choisi parmi la zircone, l'oxyde d'aluminium et des composites carbone/carbone.

6. Attache selon l'une des revendications 1, 2 ou 3, où ledit élément pénétrant dans l'os est fabriqué en métal.

7. Attache selon la revendication 6, où ledit métal est choisi parmi l'acier inoxydable, le titane, un alliage de titane et un alliage cobalt-chrome-molybdène.

8. Attache selon l'une des revendications précédentes, où ladite tige biorésorbable est fabriquée en un polymère biorésorbable choisi parmi le poly(p-dioxanone), polylactide, polyglycolide, polycaprolactone, poly(orthoésters) et le polymère de carbonate triméthylène et des copolymères et/ou des mélanges de ceux-ci.

9. Attache selon l'une des revendications précédentes, où ladite tige biorésorbable et l'élément pénétrant dans l'os sont assemblés par vissage.

10. Attache selon l'une des revendications précédentes, où ladite tige biorésorbable a un élément pénétrant dans l'os précité attaché à chacune de ses extrémités.

11. Attache selon la revendication 10, où lesdits éléments pénétrant dans l'os ont chacun une longueur d'environ 7,5 à 12,5 cm (3 à 5 pouces).
